# EUROPEAN PATENT SPECIFICATION

(11) **EP 1 312 635 B1**
(45) Date of publication and mention of the grant of the patent: **24.08.2005**
(21) Application number: 03003153.8
(22) Date of filing: 19.03.2001
(51) Int. Cl.: C08G 79/02, A61K 6/00, A61K 6/087

(54) **Dental implants having bacterial resistance**
Zahnimplantate mit antibakteriellen Eigenschaften
Implant dentaire ayant une résistance bactérienne

(30) Priority: 18.03.2000 DE 10013639
(43) Date of publication of application: 21.05.2003
(62) Divisional of application: 01915375.8
(73) Proprietor: Polyzenix GmbH, 89077 Ulm (DE)
(72) Inventor: Grunze, Michael, 69151 Neckargemünd (DE)
(74) Representative: Müller-Boré & Partner Patentanwälte

(56) References cited:
- WO-A-99/16477
- DE-A- 19 613 048
- US-A- 4 579 880
- US-A- 5 548 060
- US-A- 5 997 301
- US-A- 6 077 916
- DATABASE WPI Section Ch, Week 199521 Derwent Publications Ltd., London, GB; Class A26, AN 1995-158979 XP002236094 & JP 07 082279 A (UNIV NIPPON), 28 March 1995 (1995-03-28)
- ALLCOCK: "POLYPHOSPHAZENES" INORGANIC POLYMERS, 1992, pages 61-139, XP000866367

## Description

The present invention relates to polyphosphazene derivatives and their use, having excellent biocompatible properties and imparting bacterial resistance to a coating of an article such as a dental implant. In particular, the coating is applied on at least part of a surface of e.g. said dental implant and can be used for preventing and/or reducing an inflammatory response upon application of said dental implant to a patient.

The major complications from artificial temporary or permanent implants are, on the one hand, increased deposition of thrombocytes at the surface of the foreign body. On the other side, the behaviour towards bacterias, macrophages and proteins deposited on the surface of the implants plays an important role, since those deposits essentially lead to inflammations and other problems concemed when the implants are growing in.

One of the problems occurring is, for example, increased cell proliferation and inflammation of the injured tissue that comes into contact with the artificial implant. With vascular implants, there are not only the well-known problems of increased thrombus formation, but also restenoses (i.e. re-narrowing of the blood vessel in the region expanded by angioplasty, frequently the stent region). Those complications are initiated because of activation of the clotting and immune system by the implanted foreign object, and by damage to the vessel wall during implantation of the stent in the course of angioplasty. As a consequence, the so-called restenosis (re-narrowing of the blood vessel) is caused, and, possibly, inflammations in the treated region, so that medical and chirurgical treatment is needed promptly.

One possibility having been used to deal with those complications to prevent excessive cell proliferation, is the use of so-called covered and coated stents. A plurality of materials and also covered or coated stents usable for the preparation of such coverings or coatings are known in the prior art and have been studied. For example, WO 98/56312 describes an expandable covering made of ε-PTFE for that purpose. Other materials for that use are described in EP-A-0 810 845 in which polymers described in US-A 4,883,699 and US 4,911,691 are mentioned. Further polymers given for that purpose are, for example, hydrolyzed polyacrylonitrile (US-A 4,480,642), hydrophilic polyethers (US-A-4,798,876), and polyurethane-di-acrylates (US-A-4,424,395). Further, various hydrogels usable for that purpose are known. The number of potentially usable materials further includes poly(vinylpyrrolidone) (PVP) polymers, poly(vinyl alcohols) (PVA), poly(ethylene oxide) polymers (PEO) and poly(hydroxyethyl methacrylate) p(HEMA). Further, there are publications describing the application of a number of standard materials like polyurethanes, polyethylenes, and polypropylenes as possible materials. Mixtures of these materials are also known. A number of further materials is known from EP-A-0 804 909.

The above compounds have different properties. It can be supposed that each of the materials has specific properties for certain applications. For example, PVA dissolves in liquids very well. Other materials exhibit good blood tolerance. On the other hand, some materials are particularly well extendable. Unfortunately, however, all materials have deficiencies in certain areas. For example, PVA does not exhibit good blood tolerance. ε-PTFE can be extended very well, for example, and has also good blood tolerance, but is difficult to handle. The preparation of such coverings and coatings requires a number of processing steps (WO 96/00103). Some other materials can only be made elastically by addition of plasticizers, which reduce the blood tolerance and physical tolerance and represent a further distress for the patient due to the "flushing" of the plasticizer.

Therefore, due to the insufficient properties of the presently available materials, at present, patients are given clotting inhibitors (Vitamin K antagonists) during the postoperative treatment following angioplasty; but the dosages are problematical and show no effect conceming the inhibition of inflammation or an auto-immune response, as well as an inhibitory effect conceming restenosis.

The frequency of restenosis for the usual commercially available stents is about 30-50% within 6 months after successful angioplasty.

When using coated stents which help to avoid a reaction and especially the activation of an inflammatory response, the rate of restenoses should be decreased by preventing the cell tissue to grow into the blood vessel region. However, that technique is limited particularly by the materials and their physical and chemical properties as well as their surface properties (surface finish).

The polymeric compound poly[bis(trifluoroethoxy)phosphazene] exhibits good antithrombogenic action as a filler (see Tur, Untersuchungen zur Thrombenresistenz von Poly[bis(trifluoroethoxy)phosphazen] [Studies of resistance of poly[bis(trifluoroethoxy)phosphazene] to thrombus formation] and Hollemann Wiberg, "Stickstoffverbindungen des Phosphors" [Nitrogen compounds of phosphorus], Lehrbuch der anorganischen Chemie [Textbook of Inorganic Chemistry], 666-669, 91st-100th Edition, Walter de Gruyter Verlag, 1985; and Tur, Vinogradova et al., "Entwicklungstendenzen bei polymeranalogen Umsetzungen von Polyphosphazenen" [Trends in development of polymer-like reactions of polyphosphazenes], Acta Polymerica 39, No. 8, 424-429 (1988)). Further, polyphosphazene is used in German Patent 196 13 048 for coating artificial implants, with the intention to avoid thrombus formation on the surface of the implants, and especially heart walls can be ameliorated by this type of coating.

US-patent 5,997,301 describes a method of treating a tooth comprising providing a biodegradable polyphosphazene substance and a salt, applying the polyphosphazene substance to the tooth surface, combining a pharmacologically active substance with the polyphosphazene substance, applying the salt to the tooth surface, and reacting the polyphosphazene substance, pharmacologically active substance with the salt to form a polymeric hydrogel.

Of course, there remains the risk of inflammations as usual reaction in response to the incorporation of e.g. artificial implants into a patient, which can be only minimized by application of e.g. antibiotics. However, the severe disadvantage of bacterial multiple resistance when administering regularly antibiotics as a precaution to prevent an inflammation response is well known and is becoming more and more important. Nevertheless, even the use of antibiotics can not avoid certain auto-immune reactions caused by the devices used.

Therefore, the technical problem underlying the present invention is to provide a new system and method for dental implants which should, on one hand, impart outstanding mechanical characteristics and physically tolerated properties to the dental implants so as to improve the biocompatibility of the dental implants, and should also prevent or reduce the previously mentioned sequelae of successful treatment or implantation, and on the other hand, should exhibit, besides antithrombogenic properties, the effect of preventing or reducing inflammations and auto-immune reactions as a reaction in response to the incorporation of the foreign body into the organism, in order to reduce the dosage of or even to avoid the administration of antibiotics and to ameliorate the acceptance of a foreign device coming into contact with body tissue.

The above problem is solved by providing the embodiments characterized in the claims. In a preferred embodiment, the polymer is a biocompatible polymer which can be used for imparting bacterial resistance to the dental implant. The term "bacterial resistance" encompasses a passivating coating or surface against bacterial adhesion and/or proliferation.

The coating can be applied to any dental implant. The dental implant having the above coating on at least part of a surface of said implant, can be used for preventing and/or reducing an inflammatory response upon application of said dental implant to a patient.

The above defined polymer imparts not only biocompatibility to a coating for e.g. medical devices, but also anti-thrombogenic properties and bacterial resistance. Thus, substantially no thrombus formation, no autoimmune response and no inflammatory response of medical devices can be observed upon application to a patient. This surprising result is *inter alia* based on the fact that the blood components such as macrophages, and bacteria do not adhere and, in the case of bacteria, can not grow on the surface of said coating.

The degree of polymerization of the polymer used in the coating according to the present invention can be from 2 to ∞. However, the preferred range for the degree of polymerization is from 20 to 200,000, and more preferably 40 to 10,000,000.

Preferably, at least one of the groups R¹ to R⁶ in the polymer used is an alkoxy group substituted with at least one fluorine atom.

The alkyl groups in the alkoxy, alkylsulfonyl and dialkylamino groups are, for example, straight or branched alkyl groups with 1 to 20 carbon atoms, in which the alkyl groups can, for example, be substituted with at least one halogen atom, such as a fluorine atom.

Examples of alkoxy groups are the methoxy, ethoxy, propoxy and butoxy groups, which can preferably be substituted with at least one fluorine atom. The 2,2,2-trifluoroethoxy group is particularly preferred. Examples of alkylsulfonyl groups are methylsulfonyl, ethylsulfonyl, propylsulfonyl and butylsulfonyl groups. Examples of dialkylamino groups are dimethylamino, diethylamino, dipropylamino and dibutylamino groups.

The aryl group in the aryloxy group is, for example, a compound with one or more aromatic ring systems, in which the aryl group can, for example, be substituted with at least one alkyl group as previously defined. Examples of aryloxy groups are the phenoxy and naphthoxy groups and their derivatives.

The heterocycloalkyl group is, for instance, a ring system containing 3 to 7 atoms, with at least one ring atom being a nitrogen atom. The heterocycloalkyl group can, for example, be substituted with at least one alkyl group as previously defined. Examples of heterocycloalkyl groups are the piperidinyl, piperazinyl, pyrrolidinyl and morpholinyl groups and their derivatives. The heteroaryl group is, for example, a compound with one or more aromatic ring systems in which at least one ring atom is a nitrogen atom. The heteroaryl group can, for example, be substituted with at least one alkyl group as previously defined. Examples of heteroaryl groups are the pyrrolyl, pyridinyl, pyridinolyl, isoquinolinyl and quinolinyl groups and their derivatives.

The biocompatible coating of the dental implant according to the invention has, for example, a thickness of about 1 nm up to about 100 µm, preferably up to about 10 µm, and particularly preferably up to about 1 µm.

There is no particular limitation on the dental implant used as the substrate for the coating according to the invention and it can be any material, such as plastics, metals, metal alloys and ceramics, and the biocompatible polymer (as the matrix material or filler).

In one embodiment of the present invention, a layer containing an adhesion promoter is provided between the surface of the substrate and the biocompatible coating containing the polyphosphazene derivative of formula (I).

Preferably, the adhesion promoter or spacer, respectively, contains a polar end group. Examples are hydroxy groups, carboxy groups, carboxyl groups, amino groups or nitro groups, but end groups of the O-ED type can also be used, wherein O-ED represents an alkoxy, alkylsulfonyl, dialkylamino or aryloxy group, or a heterocycloalkyl or heteroaryl group in which nitrogen is the heteroatom, and can have different substitutents like halogen atoms, particularly fluorine atoms.

Particularly, the adhesion promoter is, for example, a silicium-organic compound, preferably an amino-terminated silane or based on aminosilane, amino-terminated alkenes, nitro-terminated alkenes and silanes, or an alkylphosphonic acid. Aminopropyltrimethoxysilane is particularly preferred.

The adhesion promoter particularly improves adhesion of the coating to the surface of a dental implant by coupling the adhesion promoter to the surface of the dental implant, for instance by ionic and/or covalent bonds and by further coupling of the adhesion promoter to reactive components, particularly to the above described polymer with the general formula (I) of the coating, for instance, through ionic and/or covalent bonds.

In one embodiment of the present invention, the dental implant having the biocompatible coating exhibits surprisingly the adhesion and/or proliferation of specific eukaryotic cells on the surface of said dental implant.

A further subject of the present invention relates to methods for preventing and/or reducing an inflammatory response upon application of a dental implant to a patient by using a coating on at least that part of the surface of the dendal implant, which comes into direct contact with tissue and/or body fluids of said patient, wherein said coating contains a biocompatible polymer having the above defined general formula (I).

### EXAMPLE 1

A 0.1 M solution of polydichlorophosphazene (0.174 g per 5 ml absolute toluene as solvent) is prepared under an inert gas atmosphere. The dental implant which was oxidatively purified, is put into this solution at room temperature for 24 h. Then, the thus immobilized polydichlorophosphazene on the artificial implant is esterified with 2,2,2-sodiumtrifluorethanolate in absolute tetrahydrofuran as solvent (8 ml absolute tetrahydrofuran, 0.23 g sodium, 1.56 ml 2,2,2-trifluorethanol). The reaction mixture is kept under the reflux for the whole reaction time. The esterification is carried out under an inert gas atmosphere at 80°C for a reaction time of 3 h. Then, the so coated substrate is washed with 4-5 ml absolute tetrahydrofuran and dried in a nitrogen stream.

After these treatments the surface was analyzed for elementary composition, stoichiometry and coating thickness using X-ray spectroscopy. The results show that all reaction steps have been successfully carried out, and that coating thicknesses of greater than 3.4 nm have been attained.

For a bacterial resistance test, the thus obtained implant is put in a petri disk which was filled with a suspension of E. coli in a nutrient solution. After 14 days incubation the artificial implant was taken off the suspension and analyzed microscopically. No adherence or growth of bacteria on the coated artificial implant could be detected.

### EXAMPLE 2

The dental implant which was oxidatively purified using Caro's acid, is immersed into an 2% solution of aminopropyltrimethoxysilane in absolute ethanol. Then, the substrate is washed with 4-5 ml absolute ethanol and kept at 105°C for 1 h in a dryer.

After coupling of aminopropyltrimethoxysilane on the oxidatively purified surface of the substrate the thus treated substrate is put into a 0.1 M solution of polydichlorophosphazene in absolute toluene for 24 h at room temperature under an inert gas atmosphere. Then, the so obtained dental implant is treated as in Example 1, resulting in a dental implant containing a coating with a thickness of < 5.5 nm.

The bacterial resistance test as described in Example 1 revealed that no adherence or growth of bacteria on the surface of the dental implant could be detected.

### EXAMPLE 3

Example 3 was carried out as described in Example 2 except that, after the coupling of aminopropyltrimethoxysilane, the dental implant is put into a 0.1 M solution of poly[bis(trifluorethoxy)phosphazene] in ethylacetate (0.121 g per 5 ml ethyl acetate) for 24 h at room temperature. Then, the thus prepared artificial implant is washed with 4-5 ml ethyl acetate and dried in a nitrogen stream.

The X-ray spectroscopy revealed that the immobilisation of poly[bis(trifluorethoxy)polyphosphazene] via aminopropyltrimethoxysilan as an adhesion promoter has been successfully carried out and coating thicknesses of < 2.4 nm.

The bacterial resistance test as described in Example 1 revealed that no adherence or growth of bacteria on the surface of the dental implant could be detected.

### EXAMPLE 4

The dental implant which was oxidatively purified with Caro's acid, is put into a 0.1 M solution of poly[bis(trifluorethoxy)phosphazene] in ethyl acetate (0.121 g per 5 ml ethyl acetate) at 70°C for 24 h. Then, the thus treated artificial implant is washed with 4-5 ml ethyl acetate and dried in a nitrogen stream.

The analysis shows that the coupling of poly[bis(trifluorethoxy)phosphazene] is successfully carried out on the surface of the dental implant, and that coating thicknesses of more < 2.1 nm have been attained.

The bacterial resistance test as described in Example 1 revealed that no adherents or growth of bacteria on the surface of the dental implant could be detected.

### EXAMPLE 5

The dental implant which was oxidatively purified with Caro's acid, is put into a melt of poly[bis(trifluorethoxy)phosphazene] at 70 °C and kept for about 10 sec up to about 10 h. Then, the thus treated dental implant is washed with 4-5 ml ethyl acetate and dried in a nitrogen stream.

The analysis shows that the coupling of poly[bis(trifluorethoxy)phosphazene] is successfully carried out on the surface of the dental implant, and that coating thicknesses of up to several millimeters have been attained.

The bacterial resistance test as described in Example 1 revealed that no adherents or growth of bacteria on the surface of the dental implant could be detected.

The dental implants containing the above defined coating and used according to the present invention surprisingly maintain the outstanding mechanical properties of the material of the dental implants. Therefore, not only the biocompatibility of dental implants can be drastically improved, but also the antithrombogenic properties can be drastically improved together with minimizing the risk of an inflammation upon application of the dental implant to a patient due to the bacterial resistance of the coating.

Moreover, the coating applied increases the chemical and physical resistance of the dental implant, which improves drastically the usage of dental implants since no depositing of salts can be observed, which does not allow adhesion of and subsequently growth of bacteria. Thus, the risk of inflammation is reduced. Further, corrosion resistance of dental implants having the biocompatible coating, can be improved.

## Claims

1. A dental implant comprising a coating which is applied on at least part of a surface of the dental implant, wherein said coating contains a polymer having the following general formula (I) 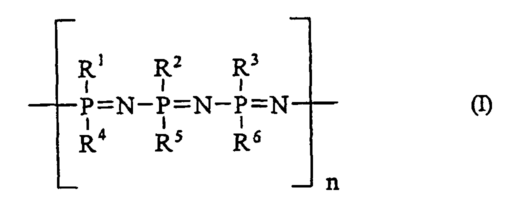 wherein
n is from 2 to ∞,
R¹ to R⁶ are the same or different and represent an alkoxy, alkylsulfonyl, dialkylamino or aryloxy group, or a heterocycloalkyl or heteroaryl group in which nitrogen is the heteroatom.

2. The dental implant according to claim 1, wherein said polymer imparts bacterial resistance to a surface of the dental implant.

## Patentansprüche

1. Zahnimplantat, umfassend eine Beschichtung, welche auf mindestens einen Teil einer Oberfläche des Zahnimplantats aufgebracht ist, wobei die Beschichtung ein Polymer mit der folgenden allgemeinen Formel (I) enthält 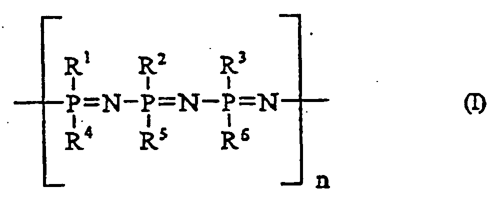 wobei
n von 2 bis ∞ ist,
R¹ bis R⁶ gleich oder unterschiedlich sind und eine Alkoxy-, Alkylsulfonyl-, Dialkylamino- oder Aryloxygruppe, oder eine Heterocycloalkyl- oder Heteroarylgruppe, in welcher Stickstoff das Heteroatom ist, darstellen.

2. Zahnimplantat nach Anspruch 1, wobei das Polymer einer Oberfläche des Zahnimplantats bakterielle Resistenz verleiht.

## Revendications

1. Implant dentaire comprenant un revêtement qui est appliqué sur au moins une partie d'une surface de l'implant dentaire, dans lequel ledit revêtement contient un polymère ayant la formule générale (I) suivante : 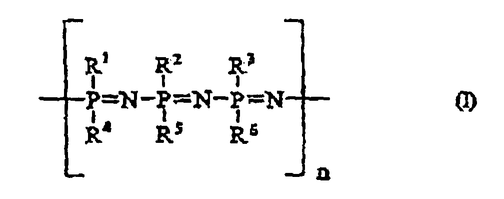 dans laquelle
n va de 2 à ∞,
R¹ à R⁶ sont identiques ou différents et représentent un groupe alcoxy, alkylsulfonyle, dialkylamino ou aryloxy, ou un groupe hétérocycloalkyle ou hétéroaryle dans lequel l'azote est l'hétéroatome.

2. Implant dentaire selon la revendication 1, dans lequel ledit polymère confère une résistance bactérienne à une surface de l'implant dentaire.
